# EUROPÄISCHE PATENTSCHRIFT

(11) **EP 0 920 289 B1**
(45) Veröffentlichungstag und Bekanntmachung des Hinweises auf die Patenterteilung: **13.11.2002**
(21) Anmeldenummer: 97918895.0
(22) Anmeldetag: 18.08.1997
(51) Int. Cl.: A61F 2/28, A61B 17/72

(54) **ENDOPROTHESE**
ENDOPROSTHESIS
ENDOPROTHESE

(30) Priorität: 16.08.1996 DE 19633865
(43) Veröffentlichungstag der Anmeldung: 09.06.1999
(73) Patentinhaber: Lob, Günter, 81377 München (DE)
(72) Erfinder: Lob, Günter, 81377 München (DE)
(74) Vertreter: Eisenführ, Speiser & Partner
(86) Internationale Anmeldenummer: DE9701819
(87) Internationale Veröffentlichungsnummer: WO98007392

(56) Entgegenhaltungen:
- EP-A- 0 281 984
- EP-A- 0 392 076
- EP-A- 0 551 611
- EP-A- 0 642 775
- EP-A- 0 649 639
- EP-A- 0 729 732
- WO-A-94/18897
- DE-A- 3 324 103
- DE-A- 3 528 728
- DE-A- 4 129 724
- US-A- 3 889 299
- US-A- 3 893 196
- US-A- 4 554 914
- US-A- 4 728 335
- US-A- 4 787 907
- US-A- 4 932 973

## Beschreibung

Die Erfindung betrifft eine Endoprothese der im Oberbegriff des ersten Anspruchs angegebenen Art, wie sie aus der Druckschrift DE-A-35 28 728 bekannt ist.

Bei ausgedehnten Knochendefekten, beispielsweise bei einer tumorbedingten Knochenresektion, besteht die Möglichkeit, durch eine den vorhandenen Knochenverlust ausgleichenden Endoprothese die entsprechenden Gliedmaßen weitestgehend in Funktion zu halten und eine ansonsten notwendige Amputation zu vermeiden.

Aus der obig genannten deutschen Offenlegungsschrift 35 28 728 ist eine Endoprothese zum Ersatz des Mittelabschnitts eines langgestreckten Knochens bekannt, welche ein hülsenförmiges Distanzstück aufweist, an dessen Enden einerseits ein feststehender Nagel und andererseits ein in dem Distanzstück axial verschieblich angeordneter Nagel vorgesehen ist.

Die vorstehend beschriebene Endoprothese weist den Nachteil auf, daß sie trotz der Zweiteiligkeit einen relativ großen Abstand zwischen den zu verbindenden Knochenfragmenten erfordert. Eine derartig weite Extension ist häufig weichteil-, muskel- oder auch sehnenbedingt nicht und wenn, dann nur mit entsprechend großer Traumatisierung realisierbar. Darüberhinaus weist die Prothese nur eine einzelne Verschraubungsmöglichkeit auf, um den axial beweglichen Nagel in einer gewählten Position gegen eine unerwünschte axiale Verschiebung zu sichern. Der zwischen der Wandung des Distanzstücks und dem in ihr verschieblich angeordneten Stift erforderliche Kraftschluß zur Sicherung der gewünschten Stiftposition ist unter Operationsbedingungen nur mit Schwierigkeiten erzeugbar.

Ausgehend von den Mängeln des Standes der Technik liegt der Erfindung deshalb insbesondere die Aufgabe zugrunde, eine Endoprothese der eingangs genannten Gattung anzugeben, welche sich einerseits durch eine große Längenvariabilität auszeichnet, operationstechnisch zu weiteren Vereinfachungen führt und mit geringerer Traumatisierung des operativ zu versorgenden Körperteils einfügbar ist.

Die Aufgabe wird, ausgehend von einer Endoprothese gemäß dem Oberbegriff des Anspruchs 1, durch dessen kennzeichnende Merkmale gelöst.

Die Erfindung schließt die Erkenntnis ein, daß die Traumatisierung eines durch Prothesenimplantation chirurgisch zu versorgenden Körpergewebes um so geringer ist, je besser die vorgesehene Endoprothese an den Einsatzort anpaßbar ist. Hierbei bieten aus mehreren Einzelteilen zusammensetzbare, größenvariable Prothesen besondere Vorteile.

Die Erfindung schließt weiterhin die Erkenntnis ein, daß bei einer stiftförmige Fixationsmittel und einen zweiteiligen, hülsenförmigen Zwischenkörper aufweisenden Endoprothese zum Ersatz von Teilen langgestreckter Knochen das Ende des rotationssymmetrischen Stiftes durch Kraftschluß - gegebenenfalls auch durch Kraft- und Formschluß - in der zweiteiligen Hülse eine axiale Verschiebung des Stiftes auch bei größerer Belastung in axialer Richtung ausgeschlossen ist. Dieser Kraftschluß wird beim Verbinden der beiden Hülsenteile, vorzugsweise durch Verschrauben, erzeugt. Vor dem Verbinden der Hülsenteile kann die Endoprothese durch ein axiales Verschieben der Fixationsmittel, welche einseitig aus dem Zwischenkörper herausragen, ohnebesonderen Aufwand in ihrer Gesamtlänge variiert werden.

Besonders angepaßte halbschalenförmige Endbereiche der Zwischenkörper mit gegenüber dem übrigen Innendurchmesser verengtem Querschnitt wirken nach Art von Spannbacken, welche stiftförmigen Fixationsmittel nach Art einer Klemme umgreifen und Arretieren, so daß die Fixationsmittel in unterschiedlicher Weise feststellbar sind. Je nach den Gegebenheiten lassen sich die Fixationsmittel in mehr oder weniger herausgeschobener Position fixieren. Dabei kann dann auch eine Rotationseinstellung vorgenommen werden, was besonders wichtig ist, wenn die Fixationsmittel gekrümmt sind und mit der Rotation eine zusätzliche Richtungseinstellung vorgenommen werden kann. Durch eine entsprechende innere Profilierung der Halbschalen ist in vorteilhafter Weise auch eine Anpassung an unterschiedliche Formquerschnitte der Fixationsmittel möglich. Diese wird unterstützt, wenn die Innenhalbschale begrenzt nachgiebig gestaltet ist, so daß mit dem Spannvorgang eine zusätzliche Formanpassung erfolgen kann, die sich in eine verbesserte Haltewirkung umsetzt.

Besondere Vorteile sind insbesondere dann erreichbar, wenn eine durch unterschiedliche Eigenschaften des für die Hülsenteile und die Stifte verwendeten Materials eine lokale Materialverformung eintritt, durch welch die an der Verankerungsstelle in Wirkungseingriff befindlichen Oberflächenabschnitte vergrößert werden.

Entsprechend der bevorzugten Ausführungsform der Erfindung weist die Endoprothese zum Ersatz knöcherner Bereiche im diaphysären und metaphysären Bereich eines langgestreckten Knochens einen in Richtung seiner Längsachse zweigeteilten Zwischenkörper auf. Der Zwischenkörper ist im wesentlichen zylindrisch ausgebildet und aus zwei gleichgeformten Halbschalen zusammensetzbar. Die Halbschalen weisen an ihren Enden jeweils eine halbzylindrische Ausnehmung auf, welche bei zusammengesetztem Zwischenkörper an beiden Enden eine Durchführung bilden. In diesen Durchführungen sind stift- oder nagelförmig ausgebildete Mittel zur intramedullären Fixation des Zwischenkörpers derart vorgesehen, daß sie in vorteilhafter Weise vor dem gegenseitigen Verbinden der Teile des Zwischenkörpers axial verschieblich in die Durchführung eingesetzt und positioniert werden können. Danach erfolgt ein Verankern der Fixationsmittel innerhalb der Durchführungen in der gewählten Position durch Verbinden der Halbschalen, wobei der erforderliche Kraftschluß vorzugsweise durch Verschrauben der Halbschalen erzeugt wird. Dadurch ist auf einfache Weise eine maßgerechte Anpassung der Endoprothese an die patientenspezifischen Bedingungen möglich, ohne daß eine übermäßige Traumatisierung des zu behandelnden Körperabschnitts eintritt.

Entsprechend der bevorzugten Ausführungsform der Erfindung ist an der Wandung der halbzylindrischen Ausnehmung an den Enden der zu dem Zwischenkörper zusammensetzbaren Teilelemente eine Profilierung vorgesehen, um eine sichere Verankerung der Fixationsmittel zu erreichen. Für diese Profilierung sind Wülste günstig, welche sich halbringförmig erstrecken und in axialer Richtung reihenförmig angeordnet sind.

Für eine mögliche Reoperation ist es entsprechend einer anderen Ausführungsform der Erfindung günstig, in die Ausnehmungen der Teilelemente jeweils einen halbzylindrischen, Muffenkörper einzulegen. Diese eingelegten halbzylindrischen Muffenkörper weisen eine Profilierung aus halbringförmigen, in axialer Richtung reihenförmig angeordneten Wülsten auf und sind bei aufgetretenem Verschleiß bequem auswechselbar. Zur Sicherung des Muffenkörpers gegen eine axiale Verschiebung ist beispielsweise in der Ausnehmung eine Ringnut vorgesehen, in welche ein an der Peripherie des Muffenkörpers angeordneter Ringwulst eingreift.

Entsprechend einer vorteilhaften Weiterbildung der Erfindung weisen sowohl die Ringwülste der Profilierung an der Innenwandung der durch die miteinander verbundenen Halbschalen des Zwischenkörpers der Endoprothese gebildeten Durchführungen als auch die Wülste der halbzylindrischen Muffenkörper ein trapez- oder dreieckförmiges bzw. ein im wesentlichen halbkreisartig ausgebildetes Querschnittsprofil auf. Dadurch ist in günstiger Weise gesichert, daß sich - in Abhängigkeit von dem Verhältnis der Festigkeit der für die Fixationsmittel und die schalenförmigen Teilelemente des Zwischenkörpers der Endoprothese verwendeten Werkstoffe - entweder eine Verformung des in einer Durchführung positionierten Schaftes eines Fixationsmittels oder eine Verformung der Ringwülste an der Wandung der Durchführung eintritt. Eine derartige Verformung führt in jedem Fall zu einer Vergrößerung der Flächenbereiche, durch welche die Einzelteile der erfindungsgemäßen Endoprothese in Wirkungseingriff stehen und damit zu einer Erhöhung des gewünschten Kraftschlusses zum Verankern der Fixationsmittel.

Um einen guten Kraft- und Formschluß zwischen Zwischenkörper und Fixationsmittel zu sichern, ist es entsprechend einer anderen Weiterbildung der Erfindung günstig, die halbringförmigen Wülste der die Durchführungen bildenden Ausnehmungen bzw. der halbzylindrischen Muffenkörper in tangentialer Richtung in einzelne Segmente zu unterteilen.

Nach einer anderen Ausführungsform der Erfindung weist der Schaft der intramedullären Fixationsmittel eine Profilierung auf, welche im wesentlichen der in den Durchführungen des Zwischenkörpers der Endoprothese vorgesehenen Profilierung entspricht. Diese Form der Fixationsmittel ermöglicht die Kombination zwischen Kraft- und Formschluß, wenn die Teilelemente des Zwischenkörpers mit eingesetzten Fixationsmitteln miteinander verbunden werden. Die Anpassung der Endoprothese an die Größe des jeweils chirurgisch zu behebenden Knochendefekts erfolgt stufenweise, wobei die Möglichkeiten einer Feinanpassung von dem Größenmaß der jeweiligen Profilierung bestimmt werden.

Unabhängig von der Gestaltung des in dem Zwischenkörper zu verankernden Schaft der Fixationsmittel sind an der Schaftperipherie Nuten vorgesehen, welche sich axial über die Gesamtlänge des Fixationsmittels erstrecken und radialsymmetrisch verteilt angeordnet sind. Die Nuten erhöhen die Verdrehsicherheit der intramedullär in die zu verbindenden Knochenabschnitte eingebrachten Fixationsmittel.

Entsprechend einer anderen vorteilhaften Ausbildungsform der erfindungsgemäßen Endoprothese sind die Fixationsmittel als hohlkörperförmige, vorzugsweise handelsübliche, Marknägel ausgebildet. Sie weisen eine Längsschlitzung auf, welche Teile der Nagelwandung erfaßt oder sich über die gesamte Nagellänge erstreckt. Der in eine Durchführung des Zwischenkörpers einsetzbare Endbereich besteht nach einer vorteilhaften Weiterbildung der Erfindung in günstiger Weise aus Vollmaterial, um eine besonders feste Verankerung im Knochenmark der zu verbindenden Knochenabschnitte zu ermöglichen. Die Längsschlitzung sichert einerseits eine radiale Elastizität und führt andererseits - begünstigt durch das Einwachsen von Knochenmaterial - zu einer ausreichenden Verdrehsicherheit der intramedullär verankerten Fixationsmittel. Zwecks Anpassung an die Lage und/oder die Form der mittels der erfindungsgemäßen Endoprothese zu einem vollständigen Knochen zu ergänzenden Knochenabschnitte ist es gegebenfalls günstig, gekrümmte Fixationsmittel einzusetzen. Von der im wesentlichen kreisbogenförmigen Krümmung sind die innerhalb des Zwischenkörpers zu verankernden Endbereiche der Fixationsmittel ausgenommen.

Um das Einwachsen der Endoprothese in dem chirurgisch behandelten Implantationsbereich zu beschleunigen, ist die äußerere Oberfläche der zu dem Zwischenkörper zusammenfügbaren Teilelemente mit einer biokompatiblen Beschichtung versehen. Zusätzlich weist die Oberfläche an den Stirnseiten der Teilelemente eine durch Noppen gebildete Narbung auf, wobei die Noppen beispielsweise halbkugelförmig ausgebildet sind. Die Wandung der Teilelemente ist mit im wesentlichen kreisförmigen Durchbrüchen versehen.

Entsprechend einer zusätzlichen Weiterbildung der Erfindung sind an den beim Zusammensetzen des Zwischenkörpers einander zugewandten Seiten der Teilelemente Rastmittel in Form von Stiften und Ausnehmungen vorgesehen, welche eine Lagesicherung der Teilelemente vor dem Verbinden ermöglichen.

Die für die erfindungsgemäß zusammensetzbar ausgebildete Endoprothese vorgesehenen Einzelteile bestehen aus einem körperverträglichen und körperbeständigen, vorzugsweise metallischen Werkstoff, beispielsweise aus Titan, Tantal, Niob oder entsprechenden Legierungen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: die bevorzugte Ausführungsform der erfindungsgemäßen Endoprothese in implantiertem Zustand,
- Figur 2a: den Zwischenkörper der in Figur 1 gezeigten Endoprothese in Ansicht von oben,
- Figur 2b: die Darstellung eines Schnittes längs der Linie A...A gemäß Figur 2a,
- Figur 2c: einen Zwischenkörper gemäß Figur 2a um 90° gedreht,
- Figur 2d: die Darstellung eines Schnittes längs der Linie B...B gemäß Figur 2c,
- Figur 2e: den in den Figuren 2a und 2c gezeigten Zwischenkörper der Endoprothese in Ansicht von vorn,
- Figur 3a: ein Teilelement des in den Figuren 2a und 2c dargestellten Zwischenkörpers in Ansicht von oben,
- Figur 3b: die Ansicht eines Schnittes längs der Linie C...C in Figur 3a,
- Figur 3c: die Ansicht eines Schnittes längs der Linie D...D in Figur 3a,
- Figur 3d: eine Darstellung der Einzelheit Z gemäß Figur 3b,
- Figuren 3e und 3f: vorteilhafte Weiterbildungen der in Figur 3d dargestellten Erfindung,
- Figur 4a: die bevorzugte Ausführungsform eines Fixationsmittels der erfindungsgemäßen Endoprothese,
- Figur 4b: das in Figur 4a gezeigte Fixationsmittel in Ansicht von links,
- Figur 4c: eine andere vorteilhafte Ausführungsform eines Fixationsmittels,
- Figur 4d: das in Figur 4ca gezeigte Fixationsmittel in Ansicht von links,
- Figur 5a: eine günstige Weiterbildung des in Figur 4a gezeigten Fixationsmittels,
- Figur 5b: die Darstellung der Ansicht eines Längsschnittes durch das Fixationsmittel gemäß Figur 5a,
- Figur 5c: die Darstellung eines Schnittes längs der Linie E...E gemäß Figur 5a,
- Figur 6a: eine weitere Ausführungsform eines Fixationsmittels für die erfindungsgemäße Endoprothese in Draufsicht,
- Figur 6b: das in Figur 6a gezeigte Fixationsmittel nach Drehung um 90° sowie
- Figur 6c: die Darstellung eines Schnittes längs der Linie F...F gemäß Figur 6b.

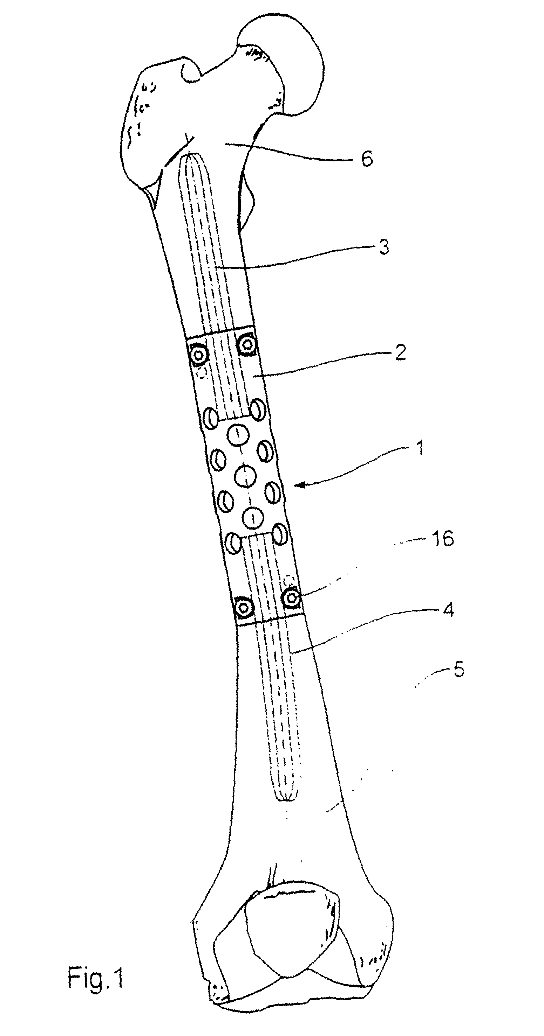

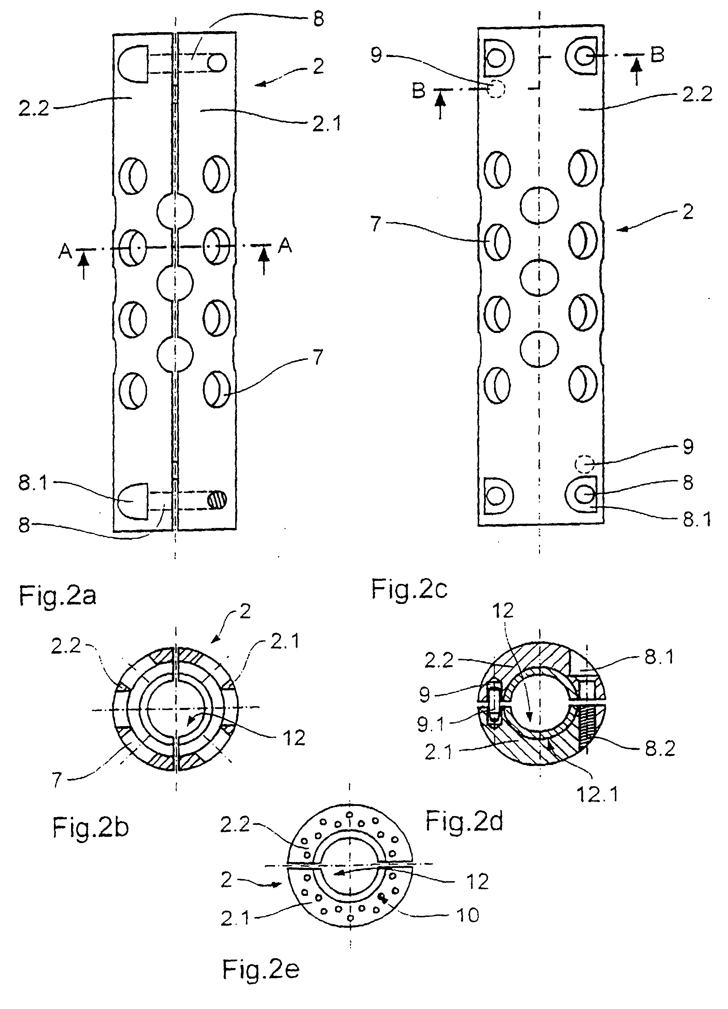

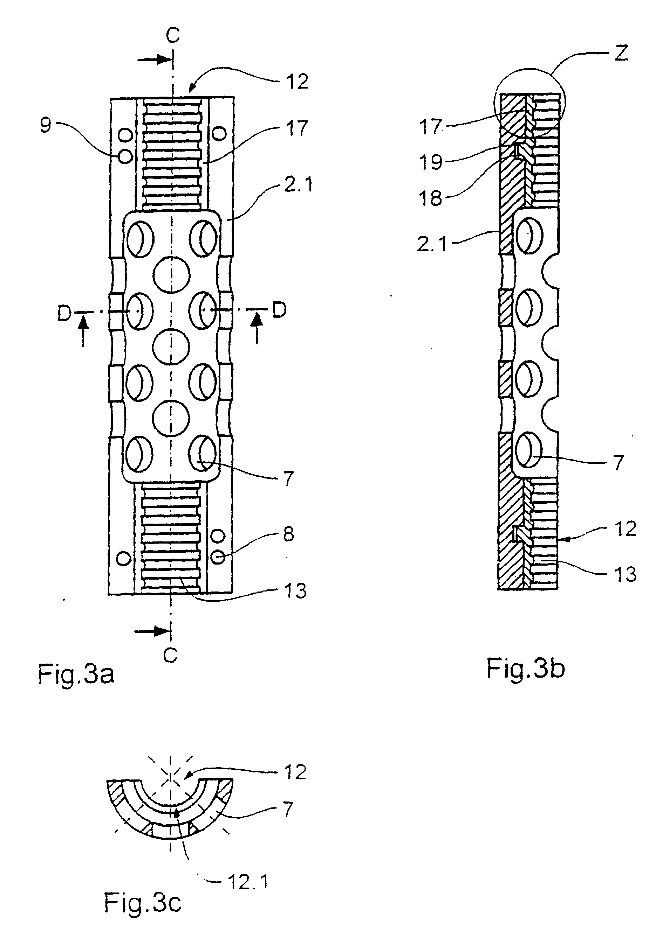

Bei dem in Figur 1 perspektivisch dargestellten Oberschenkelknochen ist der mittlere Bereich durch eine Endoprothese 1 ersetzt. Die Endoprothese 1 weist einen Zwischenkörper 2 auf, welcher einen durch Unfall oder Krankheit bedingten Knochendefekt egalisiert und die beiden Knochenabschnitte 5 und 6 miteinander verbindet. Die Verbindung zwischen den Knochenabschnitten 5 und 6 und dem Zwischenkörper 2 wird durch Fixationsmittel 3 und 4 hergestellt, welche in den Markraum der Knochenabschnitte 5 und 6 eingesetzt und danach mit dem zweiteilig ausgebildeten Zwischenkörper 2 kraft- oder kraft- und formschlüssig verbunden werden. Die Fixationsmittel 3, 4 weisen eine unterschiedliche Krümmung auf, um sich dem (nicht eingezeichneten) Markraum der Knochenabschnitte 5 und 6 optimal anpassen zu können. Die Verbindung der Einzelteile (vergleiche die Positionen 2.1 und 2.2 gemäß Figuren 2a bis 2e bzw. 3a bis 3c) des Zwischenkörpers 2 erfolgt durch Verschrauben, wobei die Fixationsmittel 3, 4 mit ihrem Schaft an jeder Seite etwa ein Drittel des inneren Hohlraums des Zwischenkörpers 2 beanspruchen. Die verwendeten selbstsichernden Schraubmittel sind mit 16 bezeichnet und werden durch einen Imbus-Schlüssel betätigt.

Der Zwischenkörper 2 der Endoprothese ist in den Figuren 2a bis 2e als Draufsicht, als Ansicht der Schnitte längs der Linien A...A bzw. B...B und als Ansicht von vorn dargestellt. Die Figuren 3a bis 3c zeigen ein Teilelement 2.1 des Zwischenkörpers 2 in Draufsicht und als Ansicht der Schnitte längs der Linien C...C bzw. D...D.

Die Teilelemente 2.1 und 2.2 des Zwischenkörpers 2 sind als 5 mindestens im wesentlichen gleichartig gestaltete Halbschalen ausgebildet. Bei bevorzugten Ausführungen können diese Halbschalen auch in der Weise formidentisch ausgestaltet sein, daß sie bei um 180° rotationssymmetrischer Ausführung einander zu einem vollständigen Hohlzylinder ergänzen, wobei die um 180° versetzt angeordneten Erhebungen des einen Teils in entsprechende Ausnehmungen des anderen Teils eingreifen und somit Führungen bilden.

Die Halbschalen 2.1 und 2.2 weisen an ihren Enden jeweils eine verengte halbzylindrische Ausnehmung 12.1 auf, welche beim zusammengesetzten Zwischenkörper 2 eine kreiszylindrische Durchführung 12 bilden. In dieser Durchführung sind halbzylindrische Muffenkörper 17 vorgesehen, welche bei Verschleiß ausgetauscht werden können. Zur axialen Lagesicherung der Muffenkörper 17 ist an deren Außenwandung ein Ringwulst 18 mit rechteckförmigem Querschnitt vorgesehen, welcher in eine entsprechende Nut 19 in der Ausnehmung 12.1 des Teilelements 2.1 eingreift.

Zum Vereinfachen des lagerichtigen Zusammensetzens des Zwischenkörpers 2 unter Operationsbedingungen weisen die Teilelemente 2.1 und 2.2 jeweils als Bohrung 9 und Zapfen 9.1 ausgebildete Rastmittel auf, welche sich in Eingriff befinden müssen, damit die in die Bohrungen 8 eingeführten Schraubmittel (vergleiche Position 16 in Figur 1) das Gewinde 8.2 sicher fassen können und die Durchführung 12 einen Kreisquerschnitt aufweist. Die Schraubmittel stützen sich bei zusammengesetztem Zwischenkörper 2 in der Ausnehmung 8.1 ab.

Die Wandung der Teilelemente 2.1 und 2.2 weist eine Mehrzahl von kreisförmigen, gleichmäßig verteilten Durchbrüchen 7 auf, durch welche in Verbindung mit einer (nicht dargestellten) biokompatiblen Oberflächenbeschichtung das Einwachsen von Bindegewebe und damit eine gute Verankerung der implantierten Endoprothese unterstützt wird. Die mit den Knochenabschnitten 5, 6 nach der Implantation in Wirkkontakt stehenden Stirnflächen der Teilelemente 2.1, 2.2 weisen eine Strukturierung mit halbkreisförmigen Noppen 10 auf, um eine Verankerung in der sich nach der Implantation bildenden Knochenmasse zu erleichtern.

Die in den Ausnehmungen 12.1 eingesetzten Muffenkörper 17 weisen an der Innenwandung eine Profilierung 13 auf, welche als halbringförmige Wülste ausgebildet ist. Diese Profilierung sichert einen guten Kontakt mit den in sie achsparallel eingesetzten Fixationsmitteln (vergleiche die Positionen 3 und 4 in Figur 1). Nach Verschrauben der Teilelemente 2.1 und 2.2 werden deren in den Durchführungen 12 befindlichen Enden durch die entstehende Flächenpressung derart kraftschlüssig verankert, daß eine axiale Verschiebung bei normaler mechanischer Belastung der Endoprothese ausgeschlossen werden kann.

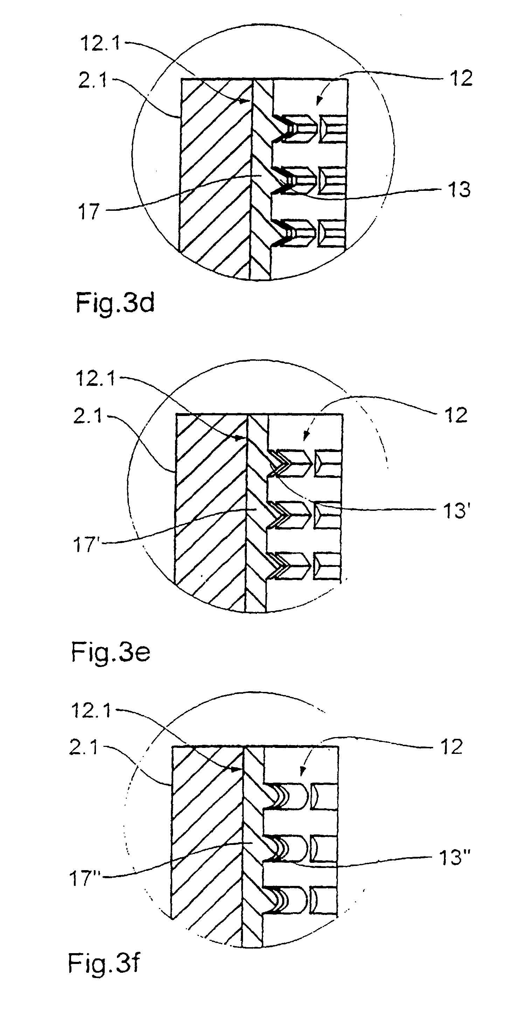

Die Figuren 3d, 3e und 3f zeigen als Einzelheit Z vorteilhafte Querschnittsprofile 13, 13' und 13" verschieden ausgebildeter Wülste der halbzylindrischen Muffenkörper 17, 17', 17". In Abhängigkeit der mechanischen Festigkeit der für die Fixationsmittel bzw. die Teilelemente des Zwischenkörpers verwendeten Werkstoffe werden bei Verschrauben der Teilelemente des Zwischenkörpers entweder die Wülste oder es wird die Mantelfläche der in die Durchführung 12 eingesetzten Fixationsmittel dahingehend verformt, daß eine vergrößerte Eingrifffläche entsteht, durch welche der erforderliche Kraftschluß zum sicheren Verankern der Fixationsmittel erzeugbar ist. Für ein derartiges Verformungsverhalten sind trapezförmige (Figur 3d), dreieckförmige (Figur 3e) oder halbkreisförmige Querschnittsprofile (Figur 3d) der Wülste günstig. Die halbringförmigen Wülste sind in tangentialer Richtung in einzelne Segmente unterteilt, um auch beispielsweise bei einer geringfügigen Achsabweichung zwischen Zwischenkörper und Fixationsmittel einen sicheren Kraft- und Formschluß zu erreichen.

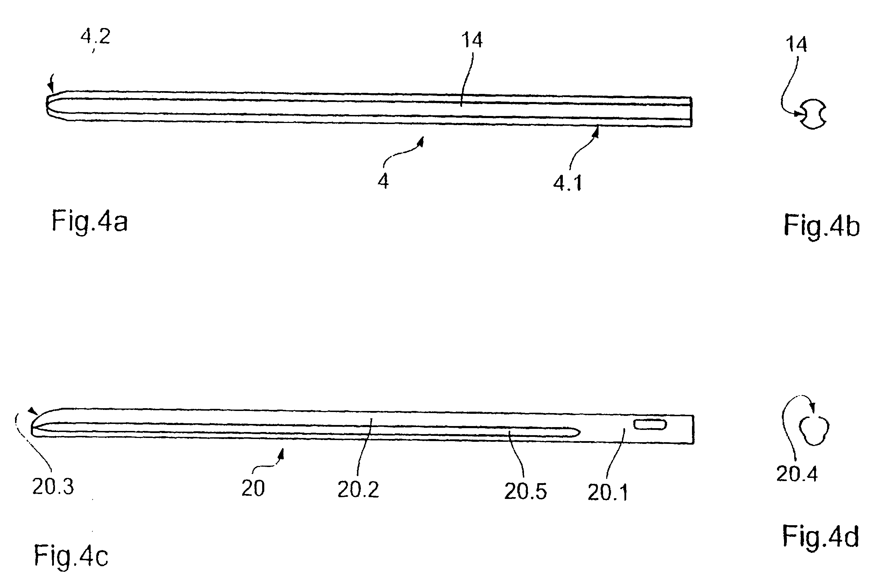

In den Figuren 4a bis 4d, 5a bis 5c und 6a bis 6c sind verschiedene Mittel zur intramedullären Fixation des Zwischenkörpers der Endoprothese in Draufsicht bzw. als Ansicht von der Seite oder als Schnittansicht dargestellt.

Das stiftförmige Fixationsmittel 4 entsprechend Figur 4a besteht aus Vollmaterial und weist zwecks Verdrehsicherung nach Implantation der Endoprothese zwei sich diametral gegenüber angeordnete Längsnuten 14 auf. Der Endbereich 4.1 wird in dem Zwischenkörper verankert. Das Stiftende 4.2 verjüngt sich und erleichtert dadurch das Einbringen des Fixationsmittels 4 in das Knochenmark.

Der Marknagel 20 gemäß Figur 4c ist als Hohlkörper mit einer einseitigen Verjüngung 20.3 und einem sich axial über die gesamte Länge erstreckenden Schlitz 20.4 ausgebildet.

Der Schlitz sichert eine radiale Federwirkung beim Einbringen in den Markraum und beim Verankern im Zwischenkörper. Die Längsnut 20.5 dient der Verdrehsicherheit nach erfolgter Implantation.

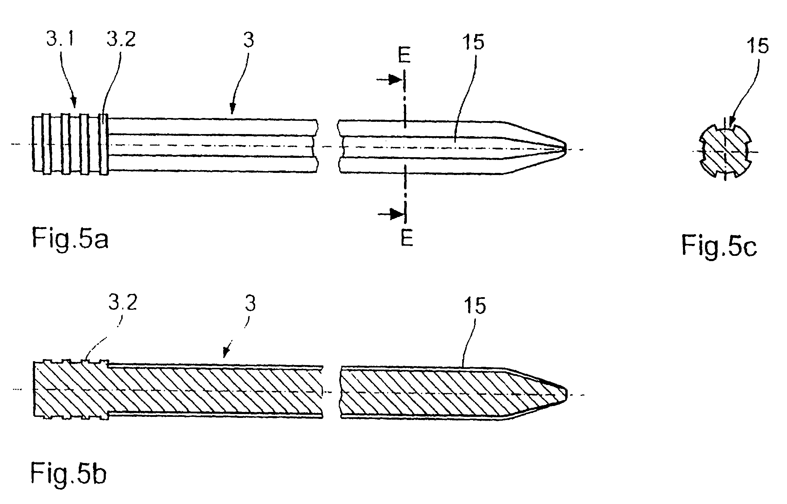

Das in den Figuren 5a bis 5c dargestellte Fixationsmittel 3 weist an seinem in dem Zwischenkörper zu verankernden Ende 3.1 eine Profilierung aus Ringwülsten 3.2 auf. Diese Profilierung stimmt mit einer in den Ausnehmungen der Teilelemente (vergleiche beispielsweise die Positionen 2.1, 12.1 und 13" gemäß Figur 3f) überein und ermöglicht eine Verankerung des profilierten Endes des Fixationsmittels durch Kraft- und Formschluß, wenn der Zwischenkörper zusammengesetzt wird. Zur Verdrehsicherheit der implantierten Endoprothese sind die vier radialsymmetrisch angeordneten Längsnuten 15 vorgesehen.

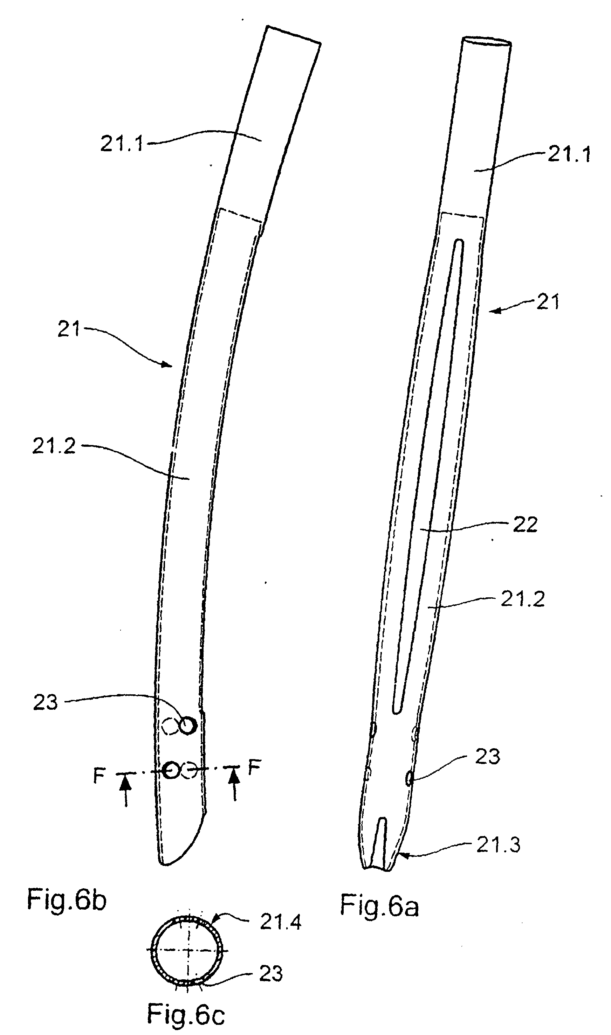

Der in den Figuren 6a bis 6c gezeigte Marknagel 21 ist als Hohlkörper ausgebildet, dessen zur Verankerung in dem Zwischenkörper vorgesehenes Ende 21.1 aus Vollmaterial besteht. Die Elastizität des hohlen Schaftes 21.2 wird durch zwei Längsnuten 22 unterstützt. Die sich verjüngende Schaftspitze 21.3 und die dort befindlichen Durchbrüche 23 erleichtert das Einführen des Fixationsmittels in den Markkanal. Die leichte Krümmung der Marknagelschaftes erleichtert die Anpassung der Endoprothese an bogenförmig verlaufende Abschnitte des Markraums von zu verbindenden Knochenstücken.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht, sofern diese Varianten in den Schutzbereich des Anspruchs 1 fallen.

## Patentansprüche

1. Endoprothese (1) zum Ersatz knöcherner Bereiche im diaphysären und metaphysären Bereich eines Knochens in Form eines Zwischenkörpers (2) mit Mitteln (3, 4, 20, 21) zu dessen intramedullärer Fixation,
**dadurch gekennzeichnet,**
**dass** die Endoprothese (1) zumindest ein separates, mit dem Zwischenkörper (2) lösbar verbindbares intramedulläres Fixationsmittel (3, 4, 20, 21) aufweist und dass der Zwischenkörper (2) in Längsrichtung teilbar ausgestaltet ist, wobei die lösbar miteinander verbundenen Teilelemente (2.1, 2.2) unter Einschluss mindestens an ihren Endbereichen vorgesehener Ausnehmungen (12.1) zumindest eine sich axial erstreckende Durchführung (12) als Halterung für das zumindest eine intramedulläre Fixationsmittel (3, 4, 20, 21), insbesondere in Form eines Knochennagels, bilden.

2. Endoprothese nach Anspruch 1, **dadurch gekennzeichnet, daß** der Zwischenkörper (2) eine im wesentlichen zylindrische Form aufweist und aus zwei im wesentlichen gleichartig ausgebildeten Halbschalen (2.1, 2.2) besteht.

3. Endoprothese nach Anspruch 1, **dadurch gekennzeichnet, daß** in jede der Durchführungen (12) ein Fixationsmittel (3, 4) mit einem seiner Enden (3.1, 4.1) arretierend einspannbar ist.

4. Endoprothese nach Anspruch 3, **dadurch gekennzeichnet, daß** die Ausnehmungen (12.1) in Form von Spannbacken ausgebildet sind.

5. Endoprothese nach Anspruch 3, **dadurch gekennzeichnet, daß** die Fixationsmittel (3, 4, 20, 21) durch Kraft- und/oder Formschluß in den Ausnehmungen der Durchführung (12) arretierbar sind.

6. Endoprothese nach Anspruch 5, **dadurch gekennzeichnet, daß** der Kraft- und Formschluß zwischen Fixationsmittel (3, 4, 20, 21) und Zwischenkörper (2) durch eine Verschraubung (16) der Teilelemente (2.1, 2.2) nach Art von Spannbacken erzeugbar ist.

7. Endoprothese nach Anspruch 1, **dadurch gekennzeichnet, daß** die Fixationsmittel (3, 4, 20, 21) stift- oder nagelförmig ausgebildet sind.

8. Endoprothese nach Anspruch 7, **dadurch gekennzeichnet, daß** die Fixationsmittel (3, 4) mindestens eine periphere, sich axial erstreckende Nut (14, 15) aufweisen.

9. Endoprothese nach Anspruch 7, **dadurch gekennzeichnet, daß** die Fixationsmittel als hohler Marknagel (20, 21) mit elastischen Wandungen (20.4, 21.4) ausgebildet ist.

10. Endoprothese nach Anspruch 9, **dadurch gekennzeichnet, daß** das in einer Durchführung (12) des Zwischenkörpers (2) zu positionierende Ende (20.1, 21.1) des Marknagels (20, 21) aus Vollmaterial besteht.

11. Endoprothese nach Anspruch 1, **dadurch gekennzeichnet, daß** die Innenwandung der die Durchführungen (12) bildenden Ausnehmungen (12.1) eine Profilierung aufweist.

12. Endoprothese nach Anspruch 1, **dadurch gekennzeichnet, daß** in die die Durchführung (12) bildenden Ausnehmungen (12.1) jeweils ein halbzylindrischer Muffenkörper (17, 17', 17") mit profilierter Innenwandung auswechselbar eingesetzt ist.

13. Endoprothese nach Anspruch 12, **dadurch gekennzeichnet, daß** der Muffenkörper (17, 17', 17") an seiner Peripherie einen Ringwulst (18) aufweist, welcher in eine ringförmige Nut (19) in der Ausnehmung (12.1) eingreift.

14. Endoprothese nach Anspruch 13, **dadurch gekennzeichnet, daß** der Ringwulst (18) einen rechteckigen Querschnitt aufweist.

15. Endoprothese nach Anspruch 11 und 12, **dadurch gekennzeichnet, daß** die Profilierung der Ausnehmung (12.1) bzw. der Innenwandung des Muffenkörpers (17, 17', 17") aus mehreren, axial versetzt angeordneten halbringförmigen Wülsten (13, 13', 13") besteht.

16. Endoprothese nach Anspruch 15, **dadurch gekennzeichnet, daß** die Wülste (13") ein bogenförmig begrenztes Querschnittsprofil aufweisen.

17. Endoprothese nach Anspruch 15, **dadurch gekennzeichnet, daß** das Querschittsprofil der Wülste (13, 13') im wesentlichen rechteck-, dreieck- oder trapezförmig ausgebildet ist.

18. Endoprothese nach Anspruch 15, 16 oder 17, **dadurch gekennzeichnet, daß** die Wülste (13, 13', 13") eine Unterteilung in tangentialer Richtung aufweisen.

19. Endoprothese nach einem der vorhergehenden Ansprüche, **dadurch gekennzeichnet, daß** die Teilelemente (2.1, 2.2) des Zwischenkörpers (2), die Muffenkörper (17, 17', 17") und die intramedullären Fixationsmittel (3, 4, 20, 21) aus Werkstoffen mit unterschiedlicher Festigkeit bestehen.

20. Endoprothese nach Anspruch 19, **dadurch gekennzeichnet, daß** die Teilelemente (2.1, 2.2) des Zwischenkörpers (2) aus einem Werkstoff bestehen, dessen Festigkeit einen geringeren Wert aufweist als die Festigkeit des für die Fixationsmittel (3, 4, 20, 21) verwendeten Werkstoffes.

21. Endoprothese nach Anspruch 19, **dadurch gekennzeichnet, daß** das mit dem Zwischenkörper (2) verbindbare Ende (3.1) des intramedullären Fixationsmittels (3) Ringwülste (3.2) aufweist, die in die Profilierung (13, 13', 13") der Durchführung (12) am proximalen oder distalen Ende des Zwischenkörpers (2) eingreifen.

22. Endoprothese nach einem der vorhergehenden Ansprüche, **dadurch gekennzeichnet, daß** an der Hüllfläche des Zwischenkörpers (2) eine biokompatible Beschichtung vorgesehen ist.

23. Endoprothese nach Anspruch 22, **dadurch gekennzeichnet, daß** die den zu verbindenden Knochen zugewandten Oberflächenabschnitte der Deckflächen der Teilelemente (2.1, 2.2) des Zwischenkörpers (2) eine Strukturierung (10) aufweisen.

24. Endoprothese nach Anspruch 23, **dadurch gekennzeichnet, daß** die Strukturierung durch im wesentlichen halbkugelförmige Noppen (10) gebildet wird.

25. Endoprothese nach einem der vorhergehenden Ansprüche, **dadurch gekennzeichnet, daß** mindestens eines der Fixationsmittel (3, 4, 20, 21) eine Krümmung aufweist.

26. Endoprothese nach Anspruch 25, **dadurch gekennzeichnet, daß** eine kreisbogenförmige Krümmung vorgesehen ist.

27. Endoprothese nach einem der vorhergehenden Ansprüche, **dadurch gekennzeichnet, daß** die zylindrische Wandung der Teilelemente (2.1, 2.2) des Zwischenkörpers (2) Durchbrüche (7) aufweist.

28. Endoprothese nach Anspruch 27, **dadurch gekennzeichnet, daß** die Durchbrüche (7) im wesentlichen kreisförmig ausgebildet sind.

## Claims

1. An endoprosthesis (1) for replacing osseous regions in the diaphysial and metaphysial region of a bone in the form of an intermediate member (2) having means (3, 4, 20, 21) for its intramedullary attachment,
**characterised in that** the endoprosthesis (1) comprises at least one separate intramedullary attachment means (3, 4, 20, 21) which can be detachably connected to the intermediate body (2) **and in that** the intermediate body (2) is divisibly constructed in the longitudinal direction, with the sub-elements (2.1, 2.2) detachably connected to one another, including recesses (12.1) provided at least at their end regions, forming at least one axially extending duct (12) as a mounting for the at least one intramedullary attachment means (3, 4, 20, 20), in particular in the form of a bone peg.

2. An endoprosthesis according to Claim 1,
**characterised in that** the intermediate member (2) has a substantially cylindrical shape and consists of two half-shells (2.1, 2.2) with a substantially similar construction.

3. An endoprosthesis according to Claim 1,
**characterised in that** an attachment means (3, 4) can be clamped and arrested by one of its ends (3.1, 4.1) in each of the ducts (12).

4. An endoprosthesis according to Claim 3,
**characterised in that** the recesses (12.1) are constructed in the form of clamping jaws.

5. An endoprosthesis according to Claim 3,
**characterised in that** the attachment means (3, 4, 20, 21) can be arrested by frictional connection and/or positive locking in the recesses of the duct (12).

6. An endoprosthesis according to Claim 5,
**characterised in that** the frictional connection and positive locking between attachment means (3, 4, 20, 21) and intermediate member (2) can be produced by a screw connection (16) of the sub-elements (2.1, 2.2) in the manner of clamping jaws.

7. An endoprosthesis according to Claim 1,
**characterised in that** the attachment means (3, 4, 20, 21) are constructed in the form of pegs or pins.

8. An endoprosthesis according to Claim 7,
**characterised in that** the attachment means (3, 4) comprise at least one peripheral, axially extending groove (14, 15).

9. An endoprosthesis according to Claim 7,
**characterised in that** the attachment means are constructed as a hollow intramedullary pin (20, 21) having elastic walls (20.4, 21.4).

10. An endoprosthesis according to Claim 9,
**characterised in that** the end (20.1, 21.1) of the intramedullary pin (20, 21) that is to be positioned in a duct (12) of the intermediate member(2) is made from solid material.

11. An endoprosthesis according to Claim 1,
**characterised in that** the inner wall of the recesses (12.1) forming the ducts (12) have a profiling.

12. An endoprosthesis according to Claim 1,
**characterised in that** in each case a semicylindrical sleeve member (17, 17;, 17") having a profiled inner wall is exchangeably inserted into the recesses (12.1) forming the duct (12).

13. An endoprosthesis according to Claim 12,
**characterised in that** the sleeve member (17, 17', 17") comprises an annular ring (18) at its periphery which engages in an annular groove (19) in the recess (12.1).

14. An endoprosthesis according to Claim 13,
**characterised in that** the annular ring (18) has a rectangular cross section.

15. An endoprosthesis according to Claim 11 and 12,
**characterised in that** the profiling of the recess (12.1) and of the inner wall of the sleeve body (17, 17', 17") consists of several axially displaced swellings (13, 13', 13").

16. An endoprosthesis according to Claim 15,
**characterised in that** the swellings (13") comprise a curved limited cross-sectional profile.

17. An endoprosthesis according to Claim 15,
**characterised in that** the cross-sectional profile of the swellings (13, 13') has a substantially rectangular, triangular or trapezoidal design.

18. An endoprosthesis according to Claim 15, 16 or 17,
**characterised in that** the swellings (13, 13', 13") are d divided in the tangential direction.

19. An endoprosthesis according to one of the preceding Claims,
**characterised in that** the sub-elements (2.1, 2.2) of the intermediate member (2), the sleeve members (17, 17', 17") and the intramedullary attachment means (3, 4, 20, 21) are made from materials having different strengths.

20. An endoprosthesis according to Claim 19,
**characterised in that** the sub-elements (2.1, 2.2) of the intermediate member (2) are made from a material whose strength has a lower value than the strength of the material used for the attachment means (3, 4, 20, 21).

21. An endoprosthesis according to Claim 19,
**characterised in that** the end (3.1) of the intramedullary attachment means (3) that can be connected to the intermediate member (2) comprises annular rings (3.2), which engage in the profiling (13, 13', 13") of the duct (12) at the proximal or distal end of the intermediate body (2).

22. An endoprosthesis according to one of the preceding Claims,
**characterised in that** a biocompatible coating is provided at the enveloping surface of the intermediate member (2).

23. An endoprosthesis according to Claim 22,
**characterised in that** the surface sections, facing the bone to be joined, of the covering surfaces of the sub-elements (2.1, 2.2) of the intermediate member (2) have a structuring (10).

24. An endoprosthesis according to Claim 23,
**characterised in that** the structuring is formed by substantially hemispherical knobs (10).

25. An endoprosthesis according to one of the preceding Claims,
**characterised in that** at least one of the attachment means (3, 4, 20, 21) has a curvature.

26. An endoprosthesis according to Claim 25,
**characterised in that** a curvature shaped as a circular arc is provided.

27. An endoprosthesis according to one of the preceding Claims,
**characterised in that** the cylindrical wall of the sub-elements (2.1, 2.2) of the intermediate member (2) comprises openings (7).

28. An endoprosthesis according to Claim 27,
**characterised in that** the openings (7) have a substantially circular design.

## Revendications

1. Endoprothèse (1) destinée à remplacer des zones osseuses dans la zone diaphysaire et métaphysaire d'un os sous la forme d'un corps intermédiaire (2) avec des moyens (3, 4, 20, 21) pour la fixation intramédullaire de celui-ci, **caractérisée en ce que** l'endoprothèse (1) présente deux moyens de fixation intramédullaires (3, 4, 20, 21) séparés, pouvant être reliés de façon amovible au corps intermédiaire (2), et **en ce que** le corps intermédiaire (2) est configuré de façon divisible dans la direction longitudinale, les éléments partiels (2.1, 2.2) reliés ensemble de façon amovible formant, en comprenant des évidements (12.1) prévus au moins à leurs zones d'extrémité, deux passages (12) s'étendant axialement en tant que fixation pour les deux moyens de fixation intramédullaires (3, 4, 20, 21), en particulier sous la forme de clous à os.

2. Endoprothèse selon la revendication 1, **caractérisée en ce que** le corps intermédiaire (2) présente une forme essentiellement cylindrique et se compose de deux semi-monocoques (2.1, 2.2) réalisées essentiellement de façon identique.

3. Endoprothèse selon la revendication 1, **caractérisée en ce que** dans chacun des passages (12), un moyen de fixation (3, 4) peut être serré par l'une de ses extrémités (3.1, 4.1) avec effet de blocage.

4. Endoprothèse selon la revendication 3, **caractérisée en ce que** les évidements (12.1) sont réalisés sous forme de mâchoires de serrage.

5. Endoprothèse selon la revendication 3, **caractérisée en ce que** les moyens de fixation (3, 4, 20, 21) peuvent être bloqués par adhérence et/ou engagement positif dans les évidements du passage (12).

6. Endoprothèse selon la revendication 5, **caractérisée en ce que** l'adhérence et l'engagement positif entre les moyens de fixation (3, 4, 20, 21) et le corps intermédiaire (2) peuvent être générés par un vissage (16) des éléments partiels (2.1, 2.2) de type mâchoires de serrage.

7. Endoprothèse selon la revendication 1, **caractérisée en ce que** les moyens de fixation (3, 4, 20, 21) sont réalisés sous forme de broches ou de clous.

8. Endoprothèse selon la revendication 7, **caractérisée en ce que** les moyens de fixation (3, 4) présentent au moins une rainure périphérique (14, 15) s'étendant axialement.

9. Endoprothèse selon la revendication 7, **caractérisée en ce que** les moyens de fixation sont réalisés comme un clou centromédullaire creux (20, 21) avec des parois élastiques (20.4, 21.4).

10. Endoprothèse selon la revendication 9, **caractérisée en ce que** l'extrémité (20.1, 21.1) du clou centromédullaire (20, 21), à positionner dans un passage (12) du corps intermédiaire (2), se compose d'un matériau plein.

11. Endoprothèse selon la revendication 1, **caractérisée en ce que** la paroi intérieure des évidements (12.1) constituant les passages (12) présente un profilage.

12. Endoprothèse selon la revendication 1, **caractérisée en ce que** dans les évidements (12.1) constituant le passage (12), respectivement un corps à manchon semi-cylindrique (17, 17', 17") avec une paroi intérieure profilée est inséré de façon échangeable.

13. Endoprothèse selon la revendication 12, **caractérisée en ce que** le corps à manchon (17, 17', 17") présente à sa périphérie un bourrelet annulaire (18) qui vient en prise dans une rainure annulaire (19) dans l'évidement (12.1).

14. Endoprothèse selon la revendication 13, **caractérisée en ce que** le bourrelet annulaire (18) présente une section transversale rectangulaire.

15. Endoprothèse selon la revendication 11 et 12, **caractérisée en ce que** le profilage de l'évidement (12.1) ou de la paroi intérieure du corps à manchon (17, 17', 17") se compose de plusieurs bourrelets semi-annulaires (13, 13', 13") agencés de façon axialement décalée.

16. Endoprothèse selon la revendication 15, **caractérisée en ce que** les bourrelets (13") présentent un profil de section transversale limité en forme d'arc.

17. Endoprothèse selon la revendication 15, **caractérisée en ce que** le profil de section transversale des bourrelets (13, 13') est réalisé essentiellement de forme rectangulaire, triangulaire ou trapézoïdale.

18. Endoprothèse selon la revendication 15, 16 ou 17, **caractérisée en ce que** les bourrelets (13, 13', 13") présentent une division dans la direction tangentielle.

19. Endoprothèse selon l'une quelconque des revendications précédentes, **caractérisée en ce que** les éléments partiels (2.1, 2.2) du corps intermédiaire (2), les corps à manchon (17, 17', 17") et les moyens de fixation intramédullaires (3, 4, 20, 21) se composent de matériaux de résistance variable.

20. Endoprothèse selon la revendication 19, **caractérisée en ce que** les éléments partiels (2.1, 2.2) du corps intermédiaire (2) se composent d'un matériau dont la résistance présente une valeur inférieure à la résistance du matériau utilisé pour les moyens de fixation (4, 4, 20, 21).

21. Endoprothèse selon la revendication 19, **caractérisée en ce que** l'extrémité (3.1) du moyen de fixation intramédullaire (3), pouvant être reliée au corps intermédiaire (2), présente des bourrelets annulaires (3.2) qui viennent en prise dans le profilage (13, 13', 13") du passage (12) à l'extrémité proximale ou distale du corps intermédiaire (2).

22. Endoprothèse selon l'une quelconque des revendications précédentes, **caractérisée en ce qu'**un revêtement biocompatible est prévu sur la surface enveloppante du corps intermédiaire (2).

23. Endoprothèse selon la revendication 22, **caractérisée en ce que** les sections de surface, orientées vers les os à relier, des surfaces de recouvrement des éléments partiels (2.1, 2.2) du corps intermédiaire (2) présentent une texture (10).

24. Endoprothèse selon la revendication 23, **caractérisée en ce que** la texture est formée par des nopes (10) essentiellement en forme de demi-cercle.

25. Endoprothèse selon l'une quelconque des revendications précédentes, **caractérisée en ce qu'**au moins l'un des moyens de fixation (3, 4, 20, 21) présente une courbure.

26. Endoprothèse selon la revendication 25, **caractérisée en ce qu'**une courbure en forme d'arc de cercle est prévue.

27. Endoprothèse selon l'une quelconque des revendications précédentes, **caractérisée en ce que** la paroi cylindrique des éléments partiels (2.1, 2.2) du corps intermédiaire (2) présente des perforations (7).

28. Endoprothèse selon la revendication 27, **caractérisée en ce que** les perforations (7) sont réalisées essentiellement de façon circulaire.
